# EUROPEAN PATENT APPLICATION

(11) **EP 3 875 153 A1**
(43) Date of publication of application: **08.09.2021**
(21) Application number: 19877845.8
(22) Date of filing: 31.10.2019
(51) Int. Cl.: A61Q 5/00, A61Q 5/12, A61K 8/34, A61K 8/40, A61K 8/894, A61K 8/898

(54) **HAIR COSMETIC AND HAIR TREATMENT METHOD USING SAME**

(30) Priority: 02.11.2018 JP 2018206973
(71) Applicant: Shiseido Company, Ltd., Chuo-ku Tokyo 104-0061 (JP)
(72) Inventor: KURASHIMA, Takumi, Tokyo 104-0061 (JP); KOJIRI, Momoka, Tokyo 104-0061 (JP)
(74) Representative: Ter Meer Steinmeister & Partner
(86) International application number: PCT/JP2019/042716
(87) International publication number: WO 2020/090953

(57) **Abstract**

The purpose of the present invention is to provide, in an emulsified base containing at least 5 mass% of water and a higher alcohol content, a hair cosmetic which imparts a desired shape, smoothness, and water repellency to hair and has excellent retention of this effect. The present invention relates to a hair cosmetic and a hair treatment method using the same, the hair cosmetic being characterized by containing: (A) a copolymer having, in a main chain thereof, a polysiloxane structure and a polyoxyalkylene structure, and having, in a side chain thereof, a trialkoxysilane group or a silanol group; (B) a surfactant containing one or two or more of (b-1) cationic surfactants in an amount of 2.0 mass% or less; (C) a higher alcohol; and (D) at least 5 mass% of water.

## Description

### Technical Field

The present invention relates to a hair cosmetic. More specifically, the present invention relates to a hair cosmetic in cream form, which is the hair cosmetic capable of imparting a desired shape, smoothness, water repellency and the like to hair and also of holding these effects for long time with a good sense of use, and a method for using the same.

### Background Art

Various hair care components such as silicones are mixed in hair washing cosmetics such as shampoos and conditioners, and hair finishing cosmetics such as hair styling agents and hair treatments. These hair care components adhere to damaged parts of hair, repair, coat the hair and thereby improving easiness in combing.

However, polymers mixed in the conventional hair cosmetics as hair care components were capable of repairing easiness in combing hair immediately after used, but most of them were tend to be easily washed away by washing and therefore they were insufficient in holding the effects for a long time or and a texture upon use.

Patent Document 1 focuses on a sense of use when rinsing and discloses a hair cosmetic with improved softness when rinsing by mixing a highly polymerized aminosilicone and the like in a gel base in which a cationic surfactant is used. However, softness of hair can be achieved immediately after such a cosmetic was used but the effect tended to be reduced by washing on and after the following day.

Patent Document 2 discloses a hair cosmetic in which a vinyl monomer having a reactive alkoxysilane group, a lipophilic vinyl monomer having an alkyl group and the like and a cationic polymer emulsion obtained by emulsion polymerizing an acrylic hydrophilic monomer using a cationic emulsifier are mixed. This cationic polymer is considered to form a solid coat when the alkoxysilane group is converted to a silanol group through hydrolysis of and form a crosslinked structure via a dehydration condensation reaction. However, the backbone of this polymer was a vinyl polymer having a polyvinyl structure, and thus a resin feel was obvious, hair was stiff and had a coarse feel, hence a sense of use was insufficient.

Patent Document 3 describes a hair treatment method which uses a composition in which a silicone polymer having an alkoxy-(aminomethyl)-silyl group a terminal is mixed. The silicone polymer used here is considered to hold a hair form for a long time and demonstrate good aesthetic characteristics. However, the composition used in this hair treatment is nonaqueous, with a mixing amount of water being limited to less than 5%, and desired effects cannot be obtained when 5% or more of water is present.

### Citation List

### Patent Document

Patent Document 1: JP-B 5655127
Patent Document 2: JP-B 4805482
Patent Document 3: JP-A 2016-525534

### Summary of Invention

### Technical Problem

Thus, an object of the present invention is to provide a hair cosmetic, which is an emulsion type containing at least 5 mass% of water and a higher alcohol, the hair cosmetic being capable of repairing hydrophobicity, easiness in combing and smoothness of damaged hair by suitably carrying out heat treatment after application of the cosmetic to hair and having good effect-holding properties for retaining the effects for a long time.

### Solution to Problem

The present inventors have found that a hair cosmetic which has good effect-holding properties and demonstrates a high water-repellent effect and a smooth texture on use can be obtained when a copolymer including a main chain having a polysiloxane structure and a polyoxyalkylene structure and a side chain having a trialkoxysilane group or a silanol group is used in combination with a predetermined amount of a cationic surfactant, whereby the present invention was accomplished.

That is, the present invention provides a hair cosmetic comprising:
(A) a copolymer including a main chain having a polysiloxane structure and a polyoxyalkylene structure and a side chain having a trialkoxysilane group or a silanol group;
(B) a surfactant containing at most 2.0 mass% of at least one (b-1) cationic surfactant;
(C) a higher alcohol; and
(D) at least 5 mass% of water.

### Advantageous Effect of Invention

The hair cosmetic according to the present invention is obtained as an emulsion type cosmetic containing water and a higher alcohol by using a copolymer including a main chain having a polysiloxane structure and a polyoxyalkylene structure and a side chain having a trialkoxysilane group or a silanol group and combining the copolymer with a predetermined amount of a cationic surfactant, whereby hydrophobicity (water repellency) is imparted to the hair surface, fast-drying and a silky feel are achieved, hair is easily arranged, heat resistance, chemical resistance and contamination resistance (pollen and the like) are improved with a good sense on use, and these effects can be held for an extended period of time.

### Description of Embodiments

Hereinafter, each of the components mixed in the hair cosmetic of the present invention is described in detail.
(A) Copolymer including a main chain having a polysiloxane structure and a polyoxyalkylene structure and a side chain having a trialkoxysilane group or a silanol group

The polymer component (component A) mixed in the hair cosmetic of the present invention is a copolymer including a main chain (backbone) having a polysiloxane structure and a polyoxyalkylene structure and a side chain having a trialkoxysilane group or a silanol group (hereinafter abbreviated as "crosslinked silicone POA copolymer").

The crosslinked silicone POA copolymer has good adhesiveness to hair due to the hydrophilicity of the polyoxyalkylene structure present in the main chain (backbone) and imparts hydrophobicity (water repellency) to the hair surface due to the polysiloxane structure present in the main chain (backbone), and has good effect-holding properties because the trialkoxysilane group or the silanol group present in the side chain crosslinks at a comparatively low temperature thereby forming a solid (or strong) coat.

The polysiloxane structure constituting the backbone of the crosslinked silicone POA copolymer of the present invention comprises polydialkylsiloxane, preferably polydimethylsiloxane, wherein a part of alkyl groups (preferably methyl groups) may be substituted with a phenyl group.

The polyoxyalkylene structure constituting the backbone of the crosslinked silicone POA copolymer of the present invention is preferably a structure including, as a repeating unit, at least one selected from the group consisting of an oxyethylene group (EO), an oxypropylene group (PO), and an oxybutylene group (BO).

The crosslinked silicone POA copolymer in the present invention preferably further includes a side chain comprising an organic group. Examples of the organic group to be the side chain include a hydrocarbon group (preferably an alkyl group such as a straight chain or branched chain alkyl group having about 1 to 30 carbon atoms, or a phenyl group) which can be optionally substituted with an amino group, a hydroxyl group, a carboxyl group, and the like. The hydrocarbon group preferably has an amino group, and a hydrogen atom of such an amino group can further be replaced with an alkyl group and the like.

The crosslinked silicone POA copolymer of the present invention preferably further includes a nitrogen atom in the backbone thereof, and the side chain preferably bonds to the nitrogen atom.

Further specific examples of the crosslinked silicone POA copolymer include polysilicone-29 (INCI name). Polysilicone-29 is defined as a complex silicone compound obtained by the reaction between a glycidopropyl-terminated dimethyl siloxane polymer and PEG-13 diglycidyl ether, diethylaminopropylamine, and aminopropyltriisopropoxysilane.

A commercial product can be used as the crosslinked silicone POA copolymer in the present invention. For example, a copolymer contained in a hair conditioning agent under the product name "Silsoft CLX-E" sold by Momentive Performance Materials Inc. can be particularly preferably used. This copolymer is a compound belonging to "Polysilicone-29." Such a copolymer has a structure in which the backbone includes a polysiloxane structure, a polyoxyalkylene structure and a nitrogen atom, a side chain has a trialkoxysilane group and a side chain comprises an organic group, wherein the polysiloxane structure includes polydimethylsiloxane, the polyoxyalkylene structure includes polyoxyethylene and polyoxyisopropylene, and the side chains bond to the nitrogen atom of the backbone. "Silsoft CLX-E" is a product containing the copolymer, dipropylene glycol and water.

A mixing amount of the crosslinked silicone POA copolymer (component A) in the hair cosmetic of the present invention based on the total cosmetic amount ranges preferably from 0.1 to 5.0 mass%, more preferably from 0.3 to 3.0 mass%, and further preferably from 0.5 to 2.0 mass%. When a mixing amount of the crosslinked silicone POA copolymer is less than 0.1 mass%, intended effects cannot be obtained, whereas when the mixing amount is more than 5.0 mass%, a sense of use may be reduced.

### (B) Surfactant

The surfactant (component B) mixed in the hair cosmetic of the present invention contains 1 or 2 or more of (b-1) a cationic surfactant.

### (b-1) Cationic surfactant

The cationic surfactant used in the present invention is not particularly limited as long as it can be used in cosmetics. Among these, quaternary ammonium salts represented by the following formula (I) are preferable. 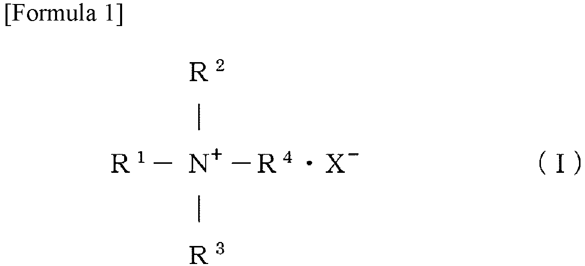 wherein R¹ is a straight chain or branched chain alkyl group having 6 to 35 carbon atoms, R², R³ and R⁴ may be the same or different and are a hydrogen atom or a straight chain or branched chain alkyl group having 1 to 3 carbon atoms, and X is a halogen ion or an organic anion.

More specifically, examples of R¹, the straight chain or branched chain alkyl group having 6 to 35 carbon atoms, include a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a ahenicosyl group, a docosyl group, a tricosyl group, a tetracosyl group, a pentacosyl group, a hexacosyl group, a heptacosyl group, an octacosyl group, nonacosyl group, a triacontyl group, a hentriacontyl group, dotriacontyl group, tritriacontyl group, tetratriacontyl group, a pentatriacontyl group, a myristoleyl group, a palmitoleyl group, an oleyl group, a linoyl group, a linoleyl group, a ricinoleyl group, and an isostearyl group. R², R³ and R⁴ may be the same or different as described above and are a hydrogen atom or a straight chain or branched chain alkyl group having 1 to 3 carbon atoms. Further, examples of X include halogen ions such as chlorine, iodine and bromine and organic anions such as methosulfate, ethosulfate, methophosphate and ethophosphate.

Examples of particularly preferable cationic surfactant in the present invention include behenyltrimethylammonium chloride (= behentrimonium chloride) wherein R¹ has 22 carbon atoms, R², R³ and R⁴ are all a methyl group, and X is chlorine, stearyltrimethylammonium chloride (= steartrimonium chloride) wherein R¹ has 18 carbon atoms, R², R³ and R⁴ are all a methyl group, and X is chlorine, and cetyltrimethylammonium chloride wherein R¹ has 16 carbon atoms, R², R³ and R⁴ are all a methyl group, and X is chlorine.

One or 2 or more of the above cationic surfactants can be mixed in combination in the hair cosmetic according to the present invention.

A mixing amount of (b-1) the cationic surfactant in the hair cosmetic of the present invention based on the total cosmetic amount is at most 2.0 mass%. When a mixing amount is more than 2.0 mass%, the holding effect of performances such as smoothness and water repellency is reduced. The lower limit value of mixing amount range is not particularly limited but is preferably 0.1 mass% or more to demonstrate superior holding effect. That is, the mixing amount range of cationic surfactant based on the total amount of the cosmetic is preferably 0.1 to 2.0 mass%, and more preferably 0.2 to 1.5 mass%.

Further, in the hair cosmetic of the present invention, a mass ratio of the crosslinked silicone POA copolymer (component A) to the cationic surfactant (component b-1), [(A)/(b-1)], is preferably 0.3 or more, and further preferably 0.4 or more. The upper limit value of this ratio is not particularly limited but is typically 20 or less, and preferably 10 or less.

The surfactant (component B) in the hair cosmetic of the present invention can include, in addition to (b-1) the cationic surfactant, (b-2) a nonionic surfactant and/or (b-3) an amphoteric surfactant.

### (b-2) Nonionic surfactant

The nonionic surfactant mixed in the hair cosmetic of the present invention is not particularly limited, and 1 or 2 or more selected from POE alkyl ethers, POE·POP alkyl ethers, POE glyceryl ether fatty acid esters, and POE castor oils or POE hydrogenated castor oils and derivatives thereof are preferably used.

For the above POE alkyl ethers and POE·POP alkyl ethers, 1 or 2 or more selected from the compounds represented by the following formula (II) and/or the following formula (III) are preferably used.

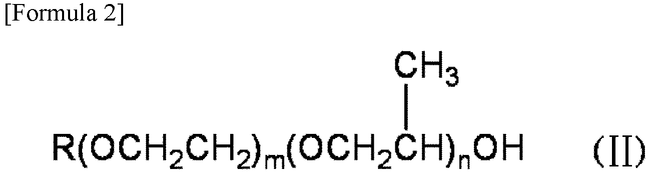

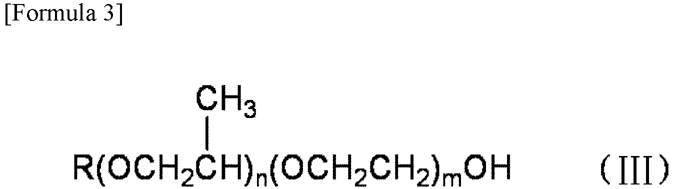

In the formulae (II) and (III), R represents an alkyl group or an alkenyl group having 12 to 24 carbon atoms, m represents a number from 5 to 30, and n represents a number from 0 to 5.

Examples of the POE alkyl ethers and POE·POP alkyl ethers include POE lauryl ether, POE cetyl ether, POE stearyl ether, POE oleyl ether, POE behenyl ether, POE decyltetradecyl ether, POE monobutyl ether, POE 2-decyltetradecyl ether, POE hydrogenated lanolin, POE glycerol ether, POE·POP lauryl ether, POE·POP cetyl ether, POE·POP stearyl ether, POE·POP oleyl ether, POE·POP behenyl ether, POE·POP decyltetradecyl ether, POE·POP monobutyl ether, POE·POP 2-decyltetradecyl ether, POE·POP hydrogenated lanolin, and POE·POP glycerol ether.

Examples of the POE glyceryl ether fatty acid esters include POE glyceryl ether monostearic acid ester, POE glyceryl ether monoisostearic acid ester, and POE glyceryl ether triisostearic acid ester.

Examples of the POE castor oils or POE hydrogenated castor oils and derivatives thereof include POE castor oils, POE hydrogenated castor oils, POE hydrogenated castor oil monoisostearates, POE hydrogenated castor oil triisostearates, POE hydrogenated castor oil monopyroglutamic acid monoisostearic acid diester, and POE hydrogenated castor oil maleic acids.

In the present invention, nonionic surfactants other than those described above can also be optionally used, and examples include POE sorbitan fatty acid esters such as POE sorbitan monooleate, and POE sorbitan tetraoleate; POE sorbit fatty acid esters such as POE sorbit monolaurate, POE sorbit monooleate, POE sorbit pentaoleate, POE sorbit monostearate; POE fatty acid esters such as POE monooleate, ethylene glycol distearate; POE alkyl phenyl ethers such as POE octyl phenyl ether, POE nonyl phenyl ether, POE dinonyl phenyl ether; pluronics such as Pluronic; tetra POE·tetra POP ethylene diamine condensates such as Tetronic; POE beeswax·lanolin derivatives such as POE sorbit beeswax; alkanolamides such as coconut fatty acid diethanolamide, lauric acid monoethanolamide, and fatty acid isopropanol amide; POE propylene glycol fatty acid esters, POE alkylamines, POE fatty acid amides, sucrose fatty acid esters, POE nonylphenyl formaldehyde condensates, alkyl ethoxy dimethylamine oxides, and trioleyl phosphoric acids.

In the present invention, nonionic surfactants having an HLB of 10 to 15 are preferably used, but not particularly limited thereto. When two or more nonionic surfactants are used, a value obtained by weightedly averaging HLB of the nonionic surfactants on the basis of HLB value and mixing amount of each nonionic surfactant is preferably 10 to 15, more preferably 11 to 14, and particularly preferably 12 to 13.

### (b-3) Amphoteric surfactant

The amphoteric surfactant used in the present invention is not particularly limited, and preferable examples include those given below.

Amide betaine amphoteric surfactants represented by the following formula (IV). For commercial products, "LEBON 2000" (manufactured by Sanyo Chemical Industries, Ltd.), "Anon B DF" (manufactured by Nippon Oil & Fats Co., Ltd.) and the like are available.

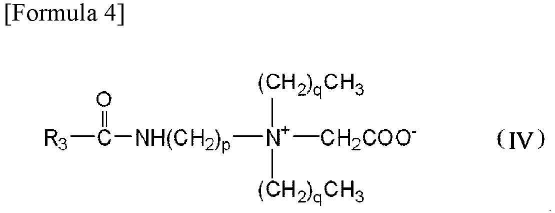

Amide sulfobetaine amphoteric surfactants represented by the following formula (V). Examples of commercial product include "Lonzaine-CS" (manufactured by Lonza), "Mirataine CBS" (manufactured by miranol), and "softazoline LHL-SF" (manufactured by Asahi Kasei Chemicals Corporation).

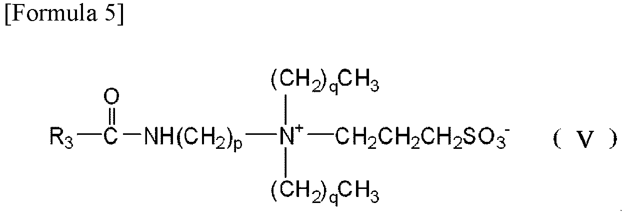

Betaine amphoteric surfactants represented by the following formula (VI). Examples of commercial product include "Anon BL" (manufactured by Nippon Oil & Fats Co., Ltd.) and "Dehyton AB-30" (manufactured by Henkel).

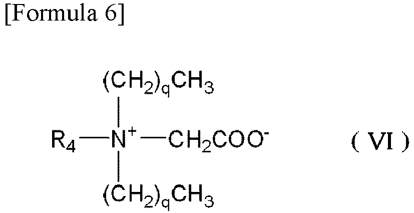

Sulfobetaine amphoteric surfactants represented by the following formula (VII). Examples of commercial product include "Lonzaine 12CS" (manufactured by Lonza).

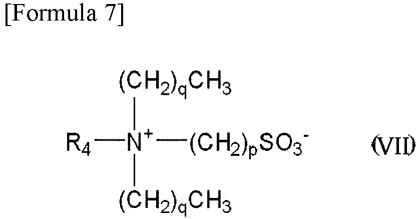

Imidazolinium amphoteric surfactants represented by the following formula (VIII). Examples of commercial product include "Obazoline 662-N" (manufactured by TOHO Chemical Industry Co., Ltd.) and "Anon GLM" (manufactured by Nippon Oil & Fats Co., Ltd.).

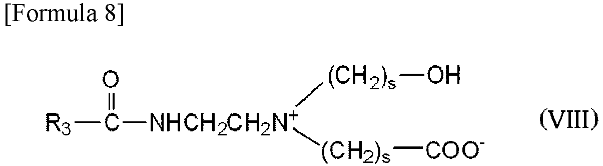

In the above formulae (IV) to (VIII), R₃ is an alkyl group or an alkenyl group having 9 to 21 carbon atoms on average, preferably an alkyl group or an alkenyl group having 11 to 17 carbon atoms on average, and more preferably an alkyl group or an alkenyl group having 11 to 13 carbon atoms on average. R₄ represents an alkyl group or an alkenyl group having 10 to 18 carbon atoms on average. p represents an integer of 2 to 4, q represents an integer of 0 to 3, and s represents an integer of 1 or 2.

Examples of the amphoteric surfactants preferably used in the present invention include alkyl(C12,14)oxyhydroxypropylarginine HCl (INCI name), and AMISAFE (registered tradename) LMA-60 (manufactured by Ajinomoto Co., Inc.) as a commercial product but are not particularly limited thereto.

The mixing amount of the nonionic surfactant (component b-2) or the amphoteric surfactant (component b-3) when mixed in the hair cosmetic of the present invention is not particularly limited but typically 0.1 to 3.0 mass%, and more preferably 0.3 to 1.5 mass%, based on the total amount of the cosmetic.

In the hair cosmetic of the present invention, when an anionic surfactant is used alone as (B) the surfactant, desired effects may not be obtained. Thus, in the hair cosmetic of the present invention, it is preferable to avoid mixing an anionic surfactant singly, and even when mixing in combination with other cationic, nonionic or amphoteric surfactants, the mixing amount thereof is preferably within the range which does not inhibit the effects of the present invention.

### (C) Higher alcohol

The higher alcohol (component C) mixed in the present invention can be those typically used for cosmetic products and pharmaceutical products. Examples include straight chain alcohols (for example, lauryl alcohol, cetyl alcohol, stearyl alcohol, behenyl alcohol, myristyl alcohol, oleyl alcohol, cetostearyl alcohol, hydrogenated rapeseed alcohol and the like); branched chain alcohols (for example, monostearyl glycerol ether (batyl alcohol), 2-decyltetradecinol, lanolin alcohol, cholesterol, phytosterol, hexyldodecanol, isostearyl alcohol, octyldodecanol and the like).

In the present invention, straight chain alcohols having 14 to 22 carbon atoms are preferably used, and straight chain alcohols having 16 to 22 carbon atoms such as stearyl alcohol, behenyl alcohol, oleyl alcohol, and cetostearyl alcohol are particularly preferably used.

In the hair cosmetic according to the present invention, higher alcohol can be mixed singly or 2 or more can be mixed in combination. A mixing amount of (C) the higher alcohol in the hair cosmetic of the present invention based on the total amount of the cosmetic can range from 0.1 to 20 mass%, and more preferably from 1.0 to 10 mass%. When a mixing amount is less than 0.1 mass%, a sense of touch when rinsing tends to be poor.

In the hair cosmetic of the present invention, a ratio of (C) the higher alcohol to (b-1) the cationic surfactant [(C)/(b-1)] is preferably 3.0 or more by mole, or 2.0 or more in terms of a mixing amount ratio. Adjustment to be such a ratio provides a hair cosmetic which is good particularly in a sense of use and effect-holding properties.

### (D) Water

The hair cosmetic of the present invention is preferably in the form of an emulsion in which the components (A) to (C) are mixed. The emulsion can be water-in-oil type, oil-in-water type or multiple emulsification type, and thus the hair cosmetic of the present invention contains water (component D) as an essential component.

Water mixed in the hair cosmetic of the present invention is not particularly limited and is preferably 5 mass% or more but typically about 50 to 90 mass% based on the total amount of the cosmetic.

In addition to the above essential components (A) to (D), other components typically used for cosmetic products and pharmaceutical products can be optionally added to the hair cosmetic of the present invention within a range which does not affect the effects of the present invention. Examples of such components includes oily components, powder components, natural polymers, synthetic polymers, thickeners, UV absorbers, sequestering agents, pH adjusting agents, skin nutrients, vitamins, antioxidants, antioxidant aids, perfumes. pH of the hair cosmetic of the present invention is preferably adjusted to 4 to 8, and more preferably 5 to 7.

The hair cosmetic of the present invention can be produced by a conventional production method commonly used for emulsion cosmetics, and is suitable to provide in the form of creams, emulsions, gels and the like.

The hair cosmetic of the present invention can be used as a cosmetic which is a type of applying to hair and "rinsing off" or a cosmetic which is a type of applying to hair and "leaving on" instead of rinsing off.

That is, the present invention provides a hair treatment method in which a moderate amount of the hair cosmetic of the present invention is applied to hair, optionally rinsed off and subsequently the hair is heat treated. The hair treatment method of the present invention, when carried out, provides hair styling and hair treatment which are good in the holding effect.

A heat treatment temperature is 40°C or more and 230°C or less, preferably 60°C or more and 180°C or less, and further preferably 80°C or more and 150°C or less. At less than 40°C, the holding effect of the effects is insufficient. At more than 230°C, hair may be damaged by heat.

### Examples

Hereinafter, the present invention will be further described in detail in reference to Examples but the present invention is not limited to these Examples. Note that the mixing amount is shown in mass% unless otherwise specified.

First, the hair cosmetic of each Example was prepared by a routine method with the formulations shown in Table 1 and Table 2 below.

Then, the hair cosmetic (0.3 g) of each Example was applied to a 15 cm (0.5 g) damaged hair strand, rinsed off with water and then heat treatment was performed at 150°C using a hair iron.

The hair strands subjected to the treatment using the hair cosmetic of each Example were evaluated for smoothness and water repellency (contact angle) by the following method/criteria.

Further, each of the hair strands was washed 5 times using a 10% SLES solution shampoo, which was free of a conditioning component, and then evaluated again for smoothness and water repellency (contact angle).

Evaluation method and evaluation criteria

### <Smoothness>

Trained researchers touched the hair strands treated with the hair cosmetic of each Example with hands and evaluate by the following evaluation criteria.

### (Evaluation criteria)

A: Hair had no coarseness or stickiness and had very smooth feeling when touched with hands.
B: Hair had no coarseness or stickiness and had smooth feeling when touched with hands.
C: Hair had rather coarseness and stickiness but had smooth feeling when touched with hands.
D: Hair had coarseness and stickiness and did not have smooth feeling when touched with hands.

### <Water repellency (contact angle)>

Two µl of water was added dropwise to the surface of the hair strand, and the water drops were photographed under a microscope. From the photographed images, left and right angles of water drops on hair were measured, and an average value thereof was defined as a single measurement value. The same measurement was repeated 3 times, and an average value thereof was defined as a value of contact angle.

**[Table 1]**

| | **Example 1** | **Example 2** | **Example 3** | **Example 4** |
|---|---|---|---|---|
| **Crosslinked silicone POA copolymer *1** | **0.75** | **0.75** | **0.75** | **0.75** |
| **Aminopropyl dimethicone** | **-** | **-** | **-** | **-** |
| **Dimethicone** | **-** | **-** | **-** | **-** |
| **Behenyltrimethylammonium chloride** | **0.4** | **0.8** | **1.6** | **0.4** |
| **Behenes-20** | **-** | **-** | **-** | **1** |
| **Sodium methyl stearoyl taurate Na** | **-** | **-** | **-** | **-** |
| **Stearyl alcohol** | **1.0** | **1.0** | **1.0** | **1.0** |
| **Behenyl alcohol** | **3.5** | **3.5** | **3.5** | **3.5** |
| **Water** | **81.15** | **80.75** | **79.95** | **80.15** |
| **Phenoxy ethanol** | **0.5** | **0.5** | **0.5** | **0.5** |
| **Perfume** | **0.5** | **0.5** | **0.5** | **0.5** |
| **Glycerol** | **10.0** | **10.0** | **10.0** | **10.0** |
| **Dipropylene glycol** | **2.0** | **2.0** | **2.0** | **2.0** |
| **Citric acid** | **0.1** | **0.1** | **0.1** | **0.1** |
| **Sodium citrate** | **0.1** | **0.1** | **0.1** | **0.1** |
| | | | | |
| **Mass ratio (A/(b-1))** | **1.9** | **0.9** | **0.5** | **1.9** |
| **Mass ratio (C/(b-1)** | **11.3** | **5.6** | **2.8** | **11.3** |
| **Molar ratio (C/(b-1))** | **14.1** | **7.1** | **3.5** | **14.1** |
| **pH** | **5.5** | **5.5** | **5.5** | **5.7** |
| | | | | |
| **Smoothness (before washing)** | **A** | **A** | **A** | **A** |
| **Smoothness (after washing 5 times)** | **A** | **A** | **A** | **A** |
| **Contact angle (before washing)** | **124.7** | **126.3** | **122.7** | **125.0** |
| **Contact angle (after washing 5 times)** | **123.5** | **118.0** | **110.8** | **122.0** |

| | | | | |
|---|---|---|---|---|
| *1: Polysilicone-29 | | | | |

**[Table 2]**

| | **Comparative Example 1** | **Comparative Example 2** | **Comparative Example 3** | **Comparative Example 4** |
|---|---|---|---|---|
| **Crosslinked silicone POA copolymer *1** | **0.75** | **0.75** | **-** | **-** |
| **Aminopropyl dimethicone** | **-** | **-** | **0.6** | **-** |
| **Dimethicone** | **-** | **-** | **0.2** | **-** |
| **Behenyltrimethylammonium chloride** | **2.4** | **-** | **1.6** | **-** |
| **Behenes-20** | **-** | **-** | **-** | **-** |
| **Sodium methyl stearoyl taurate Na** | **-** | **1** | **-** | **-** |
| **Stearyl alcohol** | **1.0** | **1.0** | **1.0** | **-** |
| **Behenyl alcohol** | **3.5** | **3.5** | **3.5** | **-** |
| **Water** | **79.15** | **80.55** | **79.90** | **100** |
| **Phenoxy ethanol** | **0.5** | **0.5** | **0.5** | **-** |
| **Perfume** | **0.5** | **0.5** | **0.5** | **-** |
| **Glycerol** | **10.0** | **10.0** | **10.0** | **-** |
| **Dipropylene glycol** | **2.0** | **2.0** | **2.0** | **-** |
| **Citric acid** | **0.1** | **0.1** | **0.1** | **-** |
| **Sodium citrate** | **0.1** | **0.1** | **0.1** | **-** |
| | | | | |
| **Mass ratio (A/ (b-1))** | **0.3** | **-** | **0** | **-** |
| **Mass ratio (C/ (b-1)** | **1.9** | **-** | **2.8** | **-** |
| **Molar ratio (C/ (b-1))** | **2.3** | **-** | **3.5** | **-** |
| **pH** | **5.5** | **5.6** | **5.8** | **-** |
| | | | | |
| **Smoothness (before washing)** | **A** | **C** | **B** | **D** |
| **Smoothness (after washing 5 times)** | **C** | **D** | **D** | **D** |
| **Contact angle (before washing)** | **124.5** | **103.0** | **120.3** | **94.3** |
| **Contact angle (after washing 5 times)** | **100.3** | **88.7** | **90.8** | **87.2** |

| | | | | |
|---|---|---|---|---|
| ***1: Polysilicone-29** | | | | |

As shown in Table 1, the hair cosmetics of Examples 1 to 4, in which (A) the crosslinked silicone POA copolymer and (b-1) 2.0 mass% or less of the cationic surfactant were combined to obtain creamy cosmetics containing (C) the higher alcohol and (D) water, well maintained smoothness and contact angles (water repellency) even after washing 5 times with the shampoo.

On the other hand, in the results shown in Table 2, Comparative Example 1 in which more than 2.0 mass% (2.4 mass%) of (b-1) the cationic surfactant was mixed had good smoothness and water repellency immediately after heat treatment but these effects were reduced after washing 5 times. Comparative Example 2 in which the anionic surfactant was mixed alone as (B) the surfactant failed to obtain sufficient properties immediately after the treatment and was equivalent to the untreated hair (Comparative Example 4) after washing 5 times. Further, Comparative Example 3 in which the conventional amino-modified silicones and the like having been commonly used in hair cosmetics were mixed in place of (A) the crosslinked silicone POA copolymer had notably reduced smoothness and water repellency after washing 5 times.

Using the hair cosmetic of Example 1, changes in properties, which were caused by the presence or absence of rinsing off after the hair cosmetic was applied and the differences in heating temperature/heating device in the case of not rinsing off, were tested. The results are shown in Table 3.

**[Table 3]**

| | **Test Example 1** | **Test Example 2** | **Test Example 3** | **Test Example 4** | **Test Example 5** |
|---|---|---|---|---|---|
| **Presence or absence of rinsing off** | **Rinsed off** | **Not rinsed off** | **Not rinsed off** | **Not rinsed off** | **Not rinsed off** |
| **Heating temperature** | **150°C** | **150°C** | **120°C** | **80°C** | **Room temperature** |
| **Heating device** | **Hair iron** | **Hair iron** | **Hair iron** | **Blow dryer** | **-** |
| | | | | | |
| **Smoothness (before washing)** | **A** | **A** | **A** | **A** | **A** |
| **Smoothness (after washing 5 times)** | **A** | **A** | **A** | **A** | **C** |
| **Contact angle (before washing)** | **124.7** | **127.0** | **124.5** | **122.3** | **118.2** |
| **Contact angle (after washing 5 times)** | **123.5** | **125.0** | **120.6** | **119.5** | **101.0** |

The presence or absence of the step of rinsing off after the hair cosmetic was applied did not have a significant impact on hair properties after heat treatment (Test Examples 1 and 2). Further, when heat temperatures were changed between 80°C and 150°C and the heat device was also changed, equivalent holding effect was obtained (Test Examples 2 to 4). However, Test Example 5, which was treated at room temperature (about 25°C), had notable property reduction after washing 5 times, thereby confirming poor holding effect.

## Claims

1. A hair cosmetic comprising:
(A) a copolymer including a main chain having a polysiloxane structure and a polyoxyalkylene structure and a side chain having a trialkoxysilane group or a silanol group;
(B) a surfactant containing 2.0 mass% or less of at least one (b-1) a cationic surfactant;
(C) a higher alcohol; and
(D) at least 5 mass% of water.

2. The hair cosmetic according to claim 1, wherein:
a mass ratio of said (A) copolymer including a main chain having a polysiloxane structure and a polyoxyalkylene structure and a side chain having a trialkoxysilane group or a silanol group to said (b-1) cationic surfactant, [(A)/(b-1)], is at least 0.3.

3. The hair cosmetic according to claim 1 or 2, wherein:
a ratio of said (C) higher alcohol to said (b-1) cationic surfactant, [(C)/(b-1)], is at least 2.0 by mass or at least 3.0 by mole.

4. The hair cosmetic according to any one of claims 1 to 3, wherein:
said polysiloxane structure constituting said (A) copolymer including a main chain having a polysiloxane structure and a polyoxyalkylene structure and a side chain having a trialkoxysilane group or a silanol group is a polydimethylsiloxane, and a part of methyl groups of said polydimethylsiloxane may be substituted with a phenyl group.

5. The hair cosmetic according to any one of claims 1 to 4, wherein:
said polyoxyalkylene structure constituting said (A) copolymer including a main chain having a polysiloxane structure and a polyoxyalkylene structure and a side chain having a trialkoxysilane group or a silanol group includes, as a repeating unit, at least one selected from the group consisting of an oxyethylene group (EO), an oxypropylene group (PO), and an oxybutylene group (BO).

6. The hair cosmetic according to any one of claims 1 to 5, wherein:
said (A) copolymer including a main chain having a polysiloxane structure and a polyoxyalkylene structure and a side chain having a trialkoxysilane group or a silanol group further includes a side chain comprising an organic group having an amino group.

7. The hair cosmetic according to any one of claims 1 to 6, wherein:
said (B) surfactant further contains, in addition to (b-1) the cationic surfactant, at least one selected from (b-2) a nonionic surfactant and (b-3) an amphoteric surfactant.

8. The hair cosmetic according to any one of claims 1 to 7, wherein:
said hair cosmetic is in cream form.

9. A hair treatment method comprising applying the hair cosmetic according to any one of claims 1 to 8 to hair, and performing heat treatment at 40°C to 230°C.
